(19) Europäisches Patentamt
European Patent Office
Office européen des brevets

(11) EP 3 378 493 B1

(12) EUROPEAN PATENT SPECIFICATION

(45) Date of publication and mention of the grant of the patent:
**13.01.2021 Bulletin 2021/02**

(21) Application number: **16865699.9**

(22) Date of filing: **09.11.2016**

(51) Int Cl.:
*A61K 9/51* (2006.01) *A61K 9/16* (2006.01)
*A61K 9/00* (2006.01) *A61K 47/34* (2017.01)
*A61K 31/337* (2006.01) *A61P 35/00* (2006.01)
*A61K 45/06* (2006.01)

(86) International application number:
**PCT/CN2016/105142**

(87) International publication number:
**WO 2017/084522 (26.05.2017 Gazette 2017/21)**

(54) **NOVEL ANTI-CANCER DRUG NANO-PREPARATION AND PREPARATION METHOD THEREFOR**

NEUARTIGES ANTIKREBSMITTEL-NANOPRÄPARAT UND HERSTELLUNGSVERFAHREN DAFÜR

NOUVELLE NANOPRÉPARATION DE MÉDICAMENT ANTICANCÉREUX ET PROCÉDÉ DE PRÉPARATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2015 CN 201510788252**

(43) Date of publication of application:
**26.09.2018 Bulletin 2018/39**

(73) Proprietors:
- **Hangzhou Push-Kang Biotechnology Co., Ltd**
**Hangzhou, Zhejiang 310030 (CN)**
- **Advanced Polymer Materials Inc.**
**Dorval, Québec (CA)**

(72) Inventors:
- **YU, Bo**
**Hangzhou**
**Zhejiang 310030 (CN)**
- **YU, Gaer**
**Hangzhou**
**Zhejiang 310030 (CN)**
- **ZHANG, Xiaomin**
**Hangzhou**
**Zhejiang 310030 (CN)**
- **ZHANG, Yingxin**
**Hangzhou**
**Zhejiang 310030 (CN)**
- **YAO, Ju**
**Hangzhou**
**Zhejiang 310030 (CN)**

(74) Representative: **Wang, Bo**
**Panovision IP**
**Ebersberger Straße 3**
**85570 Markt Schwaben (DE)**

(56) References cited:
**WO-A1-95/03356** **WO-A2-2013/160773**
**CA-C- 2 108 008** **CN-A- 102 167 794**
**CN-A- 102 772 368** **CN-A- 103 263 672**
**CN-A- 104 136 012** **US-A1- 2004 009 229**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 378 493 B1

## Description

### TECHNICAL FIELD

The present invention relates to the field of pharmaceutical formulations and, in particular, to a novel polymeric formation of a pharmaceutical composition comprising an active ingredient and a polyethylene glycol-polybutylene glycol (PEG-PBG) copolymer.

### BACKGROUND

Macromolecular carriers are a new drug delivery technology emerging with the development of pharmacological research, biomaterial science and clinical medicine. Despite a range of advantages such as high efficacy and ease of use, low molecular weight drugs tend to have relatively significant side effects. In general, a low molecular weight drug is administered orally or by injection and is metabolized rapidly in the human body with a short half-life and a lack of specificity. A macromolecular carrier is a macromolecular substance that neither has a pharmacological effect nor reacts with a low molecular weight drug that it carries either by weak hydrogen bonds formed with the drug or by connecting the drug to its polymeric main chain through a condensation polymerization reaction. In this design, the macromolecular substance can serve as a delivery system for the low molecular weight drug.

Using a macromolecular substance as a carrier for a low molecular weight drug can prolong the duration of the drug, increase its specificity and lower its toxicity. Recently, rapid development has been witnessed in macromolecular carriers of the order of micrometers or nanometers such as nanomicelles, nanovesicles and nanoparticles, etc. Such macromolecular carriers are capable of delivery and controlled release of drug molecules effectively dispersed therein in various response modes.

Nanoparticles-based drug delivery systems are developed from the application of nanotechnology and nanomaterials in the field of pharmaceuticals. Such drug delivery systems use nanoparticles (NPs) as drug carriers, which are solid, colloidal particles consisting of macromolecular substances and ranging in particle size from 10 nm to 1,000 nm. When dispersed in water, NPs can form a quasi-colloidal solution. Due to their uniqueness and superiority when used as drug carriers, NPs have become an important focus of pharmaceutical and medical research both in China and abroad.

Adjuvants used in NP formulations are mostly degradable macromolecular polymers, among which polyesters are the biodegradable macromolecular materials that have been most studied and most widely used up to now. Commonly-used polyesters are polylactic acid (PLA), polyglycolic acid (PGA), poly(lactic-co-glycolic acid) (PLGA) and polycaprolactone (PCL), etc. Macromolecular materials for the encapsulation of hydrophobic drug substances are disclosed in CN 102 167 794 A, CN 103 263 672 A, CN 104 136 012 A, and US 2004/009229 A1.

Although there have been some conventional macromolecular materials available for the preparation of nanoparticles, these materials are suffering from many disadvantages. Therefore, there is still an urgent need in this art for new macromolecular materials usable for preparing nanoparticles.

### SUMMARY

In one aspect, the present application relates to a composition

in a nanoparticle form comprising an active ingredient, which is a hydrophobic substance, and a polyethylene glycol-polybutylene glycol (PEG-PBG) copolymer of formula I, II or III

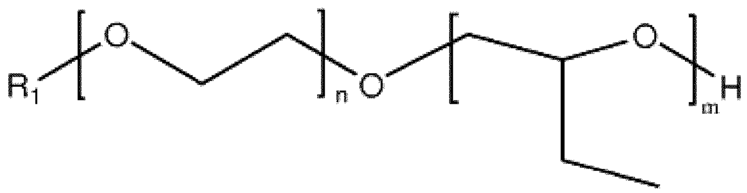

I

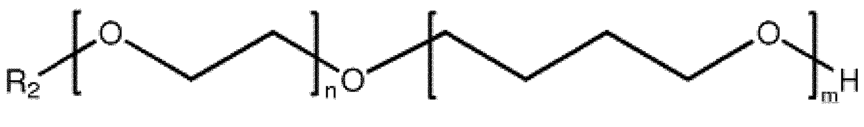

II

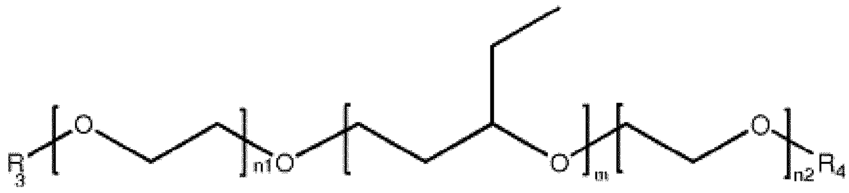

III

, in which, n, n1 and n2 each independently range from 1 to 3000, m ranges from 1 to 1500, and R1, R2, R3 and R4 are each independently H and $C_1$-$C_3$ alkyl groups.

In certain embodiments, the PEG-PBG copolymer has a molecular weight of 0.1 K-300 K.

In certain embodiments, the nanoparticles have a particle size of 10-500 nm. In certain embodiments, the nanoparticles have a particle size of 10-100 nm.

In certain embodiments, the active ingredient is selected from anti-neoplastic agents, antibiotic agents, cardiovascular agents, anti-diabetic agents and non-steroidal anti-inflammatory agents. In certain embodiments, the active ingredient is paclitaxel and its derivatives. In certain embodiments, the active ingredient is paclitaxel, docetaxel or cabazitaxel.

In certain embodiments, the active ingredient and the PEG-PBG copolymer are present in a ratio by weight of 0.01-1. In certain embodiments, the active ingredient and the PEG-PBG copolymer are present in a ratio by weight of 0.1-0.3.

In certain embodiments, the composition further comprises other polymers.

In another aspect, the present application relates to a method of preparing the composition hereof, comprising the steps of: (a) dissolving the PEG-PBG copolymer and the active ingredient in an organic solvent; (b) adding the organic phase to an aqueous solution to form an oil-water mixture; and (c) removing the organic solvent from the oil-water mixture under reduced pressure.

In certain embodiments, step (b) further includes treating the oil-water mixture with a low shear force.

In certain embodiments, the low shear force results from agitation.

In certain embodiments, the method further comprises step (d) in which the product resulting from step (c) is dried. In certain embodiments, the drying in step (d) is accomplished by lyophilization.

In certain embodiments, the organic solvent comprises tetrahydrofuran, 1,4-dioxane, dimethyl sulfoxide, acetone, N,N-dimethylmethanamide or a mixture thereof.

In certain embodiments, the organic phase and aqueous phase are present in a ratio of from 1:10 to 20:1. In certain embodiments, the organic phase and aqueous phase are present in a ratio of from 0.5:1 to 2:1.

In still another aspect, the present application relates to the use of the composition hereof in the preparation of a medicament for mitigating, treating, or preventing a disease.

In one aspect, the present relates to the use of the composition hereof in the mitigation, treatment or prevention of a disease.

In another aspect, the present disclosure relates to a method for mitigating, treating, or preventing a disease, comprising applying an effective amount of the composition hereof on a subject in need thereof.

In certain embodiments, the disease is a cancer.

In still another aspect, the present application relates to the use of a PEG-PBG copolymer in the preparation of a medicament for treating a disease.

In a further aspect, the present application relates to a method for preparing a medicament, the method comprising mixing an active ingredient with a PEG-PBG copolymer.

## BRIEF DESCRIPTION OF THE DRAWINGS

Fig. 1 shows a particle size distribution and transmission electron microscopy (TEM) image of nanoparticles.

Fig. 2 shows a comparison on *in vitro* release between Taxol and paclitaxel (PTX)-loaded EB nanoparticles.

Fig. 3 shows a comparison on pharmacokinetics between Taxol and paclitaxel (PTX)-loaded EB nanoparticles.

Fig. 4 shows cellular uptake of FITC-loaded EB nanoparticles.

Fig. 5 shows *in vivo* images of tumor-bearing nude mice administered with DiR-loaded EB nanoparticles and images of their tissues.

Fig. 6 shows variations in tumor volume and body weight of tumor-bearing nude mice respectively injected with PBS (negative control), Taxol (positive control) and paclitaxel-loaded EB nanoparticles prepared in Example 1 over time after the injection in an *in vivo* pharmacodynamic experiment.

## DETAILED DESCRIPTION

In one aspect of the present invention, a composition is provided in a nanoparticle form, comprising an active ingredient, which is a hydrophobic substance, and a polyethylene glycol-polybutylene glycol (PEG-PBG) copolymer of the afore specified formula I, II or III.

PEG-PBG Copolymer

A person of skill in the art may select the PEG-PBG copolymer of the type and characteristics that meet the practical need.

In the embodiments of the invention, the PEG-PBG copolymer is of formula I, II or III

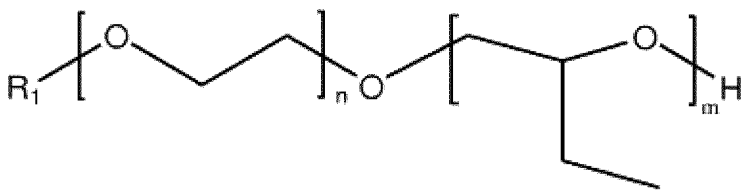

I

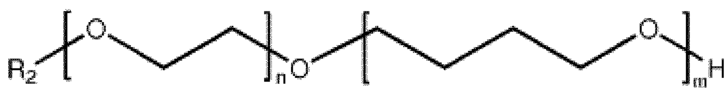

II

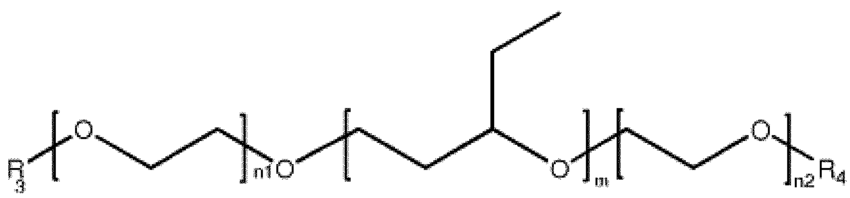

III

, in which n, n1 and n2 are each independently selected from 1-3000, m is selected from 1-1500, and $R_1$, $R_2$, $R_3$ and $R_4$ are each independently selected from H and $C_1$-$C_3$ alkyl groups. In certain embodiments, n, n1 and n2 are each independently in the range of 1-2500, 1-2000, 1-1500, 1-1200, 1-1000, 1-800, 1-600, 1-500, 1-400, 1-300, 1-200, 1-180, 1-170, 1-160, 1-150, 1-140, 1-130, 1-120, 1-118, 1-110, 1-100, 1-90, 1-80, 1-70, 1-60, 1-50, 3-2500, 5-2000, 8-1500, 10-1200, 11-800, 12-500, 13-300, 15-200, 16-180, 16-150, 16-140, 16-120, 20-100, 25-80, 30-70, 35-70 or 35-60. In certain embodiments, m is in the range of 1-1200, 1-1000, 1-800, 1-600, 1-500, 1-400, 1-300, 1-200, 1-180, 1-170, 1-160, 1-150, 1-140, 1-130, 1-120,, 1-118, 1-110, 1-100, 1-90, 1-80, 1-70, 1-60, 1-50, 3-1500, 5-1200, 8-1000, 10-800, 11-600, 12-500, 13-300, 15-200, 15-180, 15-150, 15-140, 15-120, 15-110, 15-100, 18-100, 18-90, 20-80, 25-75 or 25-70. In certain embodiments, $R_1$, $R_2$, $R_3$ and $R_4$ are each independently selected from H and $CH_3$.

In certain embodiments, the PEG-PBG copolymer has a molecular weight of 0.1 K-300 K. In certain embodiments, the molecular weight of the PEG-PBG copolymer is 0.1 K-280 K, 0.1 K-250 K, 0.1 K-200 K, 0.1 K-180 K, 0.1 K-150 K, 0.1 K-120 K, 0.1 K-100 K, 0.1 K-80 K, 0.1 K-60 K, 0.1 K-50 K, 0.1 K-40 K, 0.1 K-30 K, 0.1 K-25 K, 0.1 K-22 K, 0.1 K-20 K, 0.1 K-18 K, 0.1 K-16 K, 0.1 K-15 K, 0.1 K-14 K, 0.1 K-13 K, 0.1 K-12 K, 0.1 K-10 K, 0.1 K-8 K, 0.1 K-7 K, 0.1 K-6 K, 0.1 K-5 K, 0.1 K-4 K, 0.3 K-300 K, 0.5 K-300 K, 0.8 K-300 K, 1 K-300 K, 1.2 K-300 K, 1.2 K-250 K, 1.2 K-200 K, 1.2 K-150 K, 1.2 K-100 K, 1.2 K-80 K, 1.2 K-60 K, 1.2 K-50 K, 1.2 K-30 K, 1.2 K-20 K, 1.2 K-18 K, 1.2 K-16 K, 1.2 K-15 K, 1.2 K-14 K, 1.2 K-12 K, 1.2 K-11 K, 1.2 K-10 K, 1.2 K-8 K, 1.2 K-6 K, 1.2 K-5 K, 1.2 K-4 K, 0.5 K-150 K, 0.6 K-100 K, 0.8 K-80 K, 1 K-50 K, 1.5 K-40 K, 1.6 K-30 K, 1.7 K-20 K, 2 K-16 K, 2.5 K-14 K, 3 K-13 K, 3.5 K-12 K, 4 K-10 K or 5 K-9 K.

In this application, the molecular weight may either be a weight-average molecular weight or a number-average molecular weight. A method commonly used in the art may be employed to determine the molecular weight, such as light scattering, ultracentrifuge sedimentation or gel chromatography.

In certain embodiments, in the PEG-PBG copolymer, a molar ratio of repeating ethylene glycol units to repeating butylene glycol units ranges from 1:5 to 6:1. In certain embodiments, the molar ratio of repeated ethylene glycol units to repeated butylene glycol units in the PEG-PBG copolymer is in the range of 1:4-6:1, 1:3-6:1, 1:2-6:1, 1:1-6:1, 2:1-6:1, 3:1-6:1, 4:1-6:1, 5:1-6:1, 1:5-5:1, 1:5-4:1, 1:5-3:1, 1:5-2:1, 1:5-1:1, 1:5-1:2, 1:5-1:3, 1:4-4:1, 1:3-3:1, 1:2-3:1 or 1:1.3-2.5:1.

As used herein, the term "alkyl group", by itself of as part of another term, refers to a saturated hydrocarbon which may be straight or branched. The term "Cn-m alkyl group" refers to an alkyl group containing n to m carbon atoms. In certain embodiments, an alkyl group contains 1 to 3, or 1 to 2 carbon atoms. Examples of alkyl groups include, but are not limited to, chemical groups such as methyl, ethyl, n-propyl, isopropyl, n-butyl, tert-butyl, isobutyl and sec-butyl.

Synthesis of a polyether copolymer can be accomplished by anionic polymerization. An exemplary method for preparing the PEG-PBG copolymer according to the present application follows:

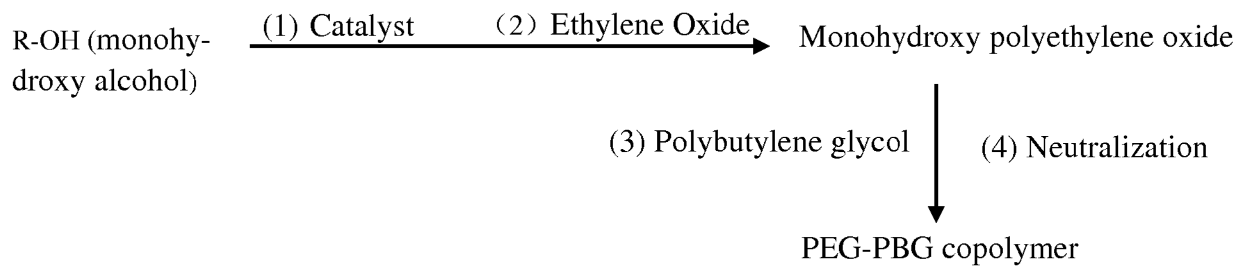


Composition

In certain embodiments, the composition hereof is a solid formulation. In certain embodiments, the composition is nanoparticles. In certain embodiments, the composition is dried nanoparticles. In certain embodiments, the composition is lyophilized nanoparticles.

In certain embodiments, the nanoparticles have a particle size of 10-500 nm. In certain embodiments, the particle size of the nanoparticles is in the range of 10-400 nm, 10-300 nm, 10-250 nm, 10-200 nm, 10-150 nm, 10-120 nm, 10-100 nm, 10-90 nm, 20-90 nm, 30-90 nm or 40-90 nm. In certain embodiments, the particle size of the nanoparticles ranges from 10 nm to 100 nm. A method commonly used in the art may be employed to determine the particle size, such as scanning electron microscopy (SEM) and light scattering. In certain embodiments, the particle size is determined by means of light scattering. In certain embodiments, the particle size is determined using a dynamic laser scatterometer.

The nanoparticles according to the present application have an acceptable coefficient of dispersion. In certain embodiments, the coefficient of dispersion of the nanoparticles according to the application is not greater than 0.3, 0.2, 0.19, 0.18, 0.17, 0.16, 0.15, 0.14, 0.13, 0.12 or 0.11. In certain embodiments, the coefficient of dispersion of the nanoparticles according to the application ranges from 0.1 to 0.2.

It will be appreciated by those skilled in the art that the composition hereof may be further modified. In certain embodiments, the composition hereof may be provided with a further encapsulation for, for example, sustained or controlled release. In certain embodiments, the composition hereof may be surface-modified with targeting groups (e.g., antibodies, ligands, specific substrates, etc.) or other macromolecules for further improving the targeting properties or kinetic parameters of the composition hereof, or for traceability of the composition hereof.

It will be appreciated by those skilled in the art that, the composition further comprises other pharmaceutically acceptable ingredients, in addition to the active ingredient and the PEG-PBG copolymer. In certain embodiments, the other ingredients include a surfactant which may be a cationic surfactant, an anionic surfactant or a non-ionic surfactant. In certain embodiments, the other ingredients include a lyopro-tectant including, but not limited to, lactose, mannose, dextran, sucrose and glycine. In certain embodiments, the other ingredients include a solution including, but not limited to, a sodium chloride solution, a glucose solution, a PBS buffer, an ethanol solution or the like.

As used herein, the term "pharmaceutically acceptable" refers to compounds, materials, compositions and/or formulations that are within the scope of proper medicinal assessment, suitable for use in contact with patient tissues, without undue toxicity, irritation, allergic response or other issues or complications, commensurate with a reasonable benefit/risk ratio and effective for the intended use.

The composition hereof is suitable to be administered by any appropriate route, for example, orally (including buccally or sublingually), rectally, nasally, topically (including buccally, sublingually or transdermally), vaginally or

parenterally (including by subcutaneous, intradermic, intramuscular, intra-articular, intra-synovial, intrasternal, intrathecal, intralesional, intravenous or subdermal injection or infusion). In certain embodiments, the composition hereof is administered parenterally. In certain embodiments, the composition hereof is administered by intravenous infusion.

Active ingredient

A person of skill in the art may properly select the active ingredient according to the practical need. active ingredient is a hydrophobic substance.

As used herein, the term "hydrophobic substance" means a substance of which less to 1 g, 0.1 g, 0.01 g, 1 mg or 0.5 mg is soluble in 100 g of water at 25.

In certain embodiments, the active ingredient is selected from anti-neoplastic agents, antibiotic agents, cardiovascular agents, anti-diabetic agents and non-steroidal anti-inflammatory agents. Illustrative examples of the active ingredient according to the present application may be: anti-neoplastic agents, such as paclitaxel, docetaxel, cabazitaxel, 5-fluorouracil, etoposide, phenylalanine mustard, chlorambucil, hexamethylmelamine, methotrexate, methyl-CCNU, vinorelbine, teniposide, homoharringtonine, hydroxycamptothecin, etc.; antibiotic agents, such as chloramphenicol, erythromycin, erythromycin estolate, erythromycin ethylsuccinate, midecamycin, josamycin, clarithromycin, rokitamycin, sulfadiazine, trimethoprim, furantoin, rifampicin, rifaximin, rifandin, dapsone, acedapsone, miconazole, etc.; cardiovascular agents, such as nifedipine, nicardipine, nitrendipine, nilvadipine, cinnarizine, perhexiline, molsidomine, digitoxin, digoxin, lanatoside C, deslanoside, propafenone, amiodarone, nitroglycerin, pentaerithrityl tetranitrate, cyclandelate, tocopherol nicotinate, etc.; anti-diabetic agents, such as tolbutamide, glibenclamide, glipizide, etc.; and non-steroidal anti-inflammatory agents, such as clemastine, cyproheptadine, pizotifen, ketotifen, tranilast, etc. Reference can be made for the structures of the particular drugs disclosed above to the instructions thereof approved by drug administrations in different countries or regions, for example, those approved by the China Food and Drug Administration, U.S. Food and Drug Administration, Japanese Pharmaceuticals and Medical Devices Agency or European Medicines Agency.

In certain embodiments, the active ingredient is paclitaxel and its derivatives. In certain embodiments, the active ingredient is paclitaxel, docetaxel or cabazitaxel (7β, 10β-dimethoxydocetaxel) and derivatives thereof.

The compounds described herein also include their salts, esters, mesomeric, racemic and isomeric forms. The isomers mentioned herein include both cis-trans and optical isomers.

As used herein, the term "derivative" means a compound resulting from the replacement of an atom or a group of atoms in a parent compound molecule by another atom or group of atoms. The derivatives of paclitaxel include, but are not limited to, its derivatives with succinic and glutaric acids, sulfonates, amino acid derivatives, phosphates, organic acid esters and carbonates, N-methyl pyridinium salts, and derivatives with polyethylene glycol, polymethacrylic acid and polyglutamic acid or polyaspartic acid.

Composition Ratio

A person of skill in the art may select a ratio of the active ingredient to the polymer according to the practical need. In certain embodiments, the ratio, by weight, of the active ingredient to the PEG-PBG copolymer, present in the composition, ranges from 0.01 to 1. In certain embodiments, the ratio, by weight, of the active ingredient to the PEG-PBG copolymer, in the composition, is in the range of 0.02-1, 0.03-1, 0.04-1, 0.05-1, 0.06-1, 0.08-1, 0.09-1, 0.1-1, 0.2-1, 0.3-1, 0.4-1, 0.5-1, 0.6-1, 0.7-1, 0.8-1, 0.9-1, 0.01-0.9, 0.01-0.8, 0.01-0.7, 0.01-0.6, 0.01-0.5, 0.01-0.4, 0.01-0.3, 0.01-0.2, 0.01-0.1, 0.01-0.09, 0.01-0.08, 0.01-0.07, 0.01-0.06, 0.01-0.05, 0.01-0.04, 0.01-0.03, 0.01-0.02, 0.03-0.9, 0.04-0.6, 0.04-0.5, 0.04-0.2, 0.04-0.1, 0.04-0.09, 0.04-0.08, 0.04-0.07, 0.04-0.06 or 0.04-0.05.

Beneficial Effects

Without wishing to be bound by theory, according to the present application, the active ingredient is encapsulated in the PEG-PBG copolymer serving as a matrix in the form of spherical particles or particles of another shape. Compared with non-nanoparticle formulations or nanoparticles formulations formed with other macromolecular materials, of the active ingredient, using the PEG-PBG copolymer as the matrix results in one or more of the following advantages: 1) prevented precipitation of the active ingredient; 2) particles with a smaller particle size; 3) higher monodispersity; 4) simpler preparation; 5) higher encapsulation efficiency; 6) better targeting; 7) a longer circulation time; 8) higher efficacy; and 9) a higher load of the active ingredient.

In another aspect of the present invention, there is provided a method for preparing the composition of the invention.

Steps of Method for Preparation

In certain embodiments, the method for preparing the composition of the invention include the steps of: (a) dissolving the PEG-PBG copolymer and the active ingredient in an organic solvent; (b) adding the organic phase to an aqueous solution to form an oil-water mixture; and (c) removing the organic solvent from the oil-water mixture under reduced pressure.

In certain embodiments, the method further include step (d) in which the product resulting from step (c) is dried. A person of skill in the art may select a proper drying process based on the specific conditions, for example, lyophilization, spray drying, etc. In certain embodiments, the drying in step (d) is accomplished by lyophilization.

(a) Dissolution of PEG-PBG Copolymer and Active Ingredient in Organic Solvent

A person of skill in the art may properly select the organic solvent based on the solubility of the active ingredient and the requirements of the preparation process. In certain embodiments, the organic solvent includes tetrahydrofuran, 1,4-dioxane, dimethyl sulfoxide, acetone, N,N-dimethylmethanamide or a mixture thereof. In certain embodiments, the organic solvent is acetone.

(b) Formation of Oil-Water Mixture by Addition of Organic Phase to Aqueous Solution

In certain embodiments, step (b) further includes treating the oil-water mixture with a low shear force.

According to the present application, the low shear force may be provided by agitation, shearing or homogenization, provided that the shear force is not greater than a shear force generated by mechanical agitation at 1000 rpm, 800 rpm, 700 rpm, 600 rpm, 500 rpm or 400 rpm. In certain embodiments, the low shear force results from agitation. In certain embodiments, the low shear force results from mechanical agitation. In certain embodiments, the agitation is performed at a speed of 100-1000 rpm, 100-800 rpm, 100-700 rpm, 100-600 rpm, 100-500 rpm or 100-400 rpm.

In certain embodiments, a ratio of the organic phase to the aqueous phase is in the range of 1:10-20:1, 1:10-18:1, 1:10-15:1, 1:10-12:1, 1:10-10:1, 1:10-8:1, 1:10-5:1, 1:10-3:1, 1:10-2:1, 1:10-1:1, 1:8-1:1, 1:6-1:1, 1:4-1:1, 1:3-1:1, 1:2.5-1:1, 1:2-1:1, 1:8-20:1, 1:6-20:1, 1:4-20:1, 1:2-20:1, 1:1-20:1, 2:1-20:1, 4:1-20:1, 5:1-20:1, 8:1-20:1, 10:1-20:1, 15:1-20:1, 1:18-20:1, 1:8-15:1, 1:6-12:1, 1:5-10:1, 1:4-8:1, 1:3-5:1, 1:3-2:1, 1:3-1:1, 1:2.5-1:1.5, 1:2.3-1:1.8 or 1:2.1-1:1.9.

(c) Removal of Organic Solvent from Oil-Water Mixture under Reduced Pressure

According to the present application, the removal under reduced pressure may be accomplished by in any suitable manner known in the art, such as rotary evaporation or drying under reduced pressure. In certain embodiments, the organic solvent is removed by rotary evaporation under reduced pressure. In certain embodiments, the rotary evaporation under reduced pressure is conducted at a vacuum degree of less than 0.6 atmosphere (atm), 0.5 atm, 0.4 atm, 0.3 atm, 0.2 atm or 0.1 atm. In certain embodiments, the vacuum degree at which the rotary evaporation under reduced pressure is in the range of 0.1-0.6 atm, 0.1-0.5 atm, 0.1-0.4 atm, 0.1-0.3 atm or 0.1-0.2 atm.

Encapsulation Efficiency

A method commonly used in the art may be employed to determine the encapsulation efficiency, such as sephadex gel filtration, ultracentrifugation or dialysis. In certain embodiments, dialysis is used to determine the encapsulation efficiency.

In certain embodiments, the encapsulation efficiency of the composition prepared by the method of the present application is not less than 80%, 83%, 85%, 87%, 89%, 90%, 92%, 93%, 94%, or 95%..

Use in Preparation of Medicament, Method for Treating Disease and Use in Treatment

In one aspect, the present application relates to the use of the composition hereof in the preparation of a medicament for mitigating, treating, or preventing a disease.

In another aspect, the present application relates to the use of the composition hereof in the mitigation, treatment or prevention of a disease.

In still another aspect, the present application relates to a method for mitigating, treating, or preventing a disease, comprising applying an effective amount of the composition hereof on a subject in need thereof.

In certain embodiments, the disease is a cancer.

"Mitigation", "treatment" or "prevention" of a disease or condition include preventing or alleviating a condition, slowing the onset or rate of development of a condition, reducing the risk of developing a condition, preventing or delaying

the development of symptoms related to a condition, reducing or ending symptoms related to a condition, generating a complete or partial regression of a condition, curing a condition, or some combination thereof.

As used in herein, the term "effective amount" refers to a quantity that can effectuate the treatment of a disease or condition in a subject or can preventively inhibit or prevent the occurrence of a disease or condition. An effective amount relieves to some extent one or more diseases or conditions in a subject, returns to normality, either partially or completely, one or more physiological or biochemical parameters causative of a disease or condition, and/or can lower the likelihood of occurrence of a disease or condition.

The effective dosage of the composition provided herein will depend on various factors known in the art, such as, for example, body weight, age, past medical history, present medications, state of health of the subject and potential for cross-reaction, allergies, sensitivities and adverse side-effects, as well as the administration route and extent of disease development. Dosages may be proportionally reduced or increased by one of ordinary skill in the art (e.g., physician or veterinarian) as indicated by these and other circumstances or requirements.

In certain embodiments, the composition provided herein may be administered at a therapeutically effective dosage ranging from about 0.01 mg/kg to about 100 g/kg (e.g., about 0.01 mg/kg, about 0.5 mg/kg, about 1 mg/kg, about 2 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, about 100 mg/kg, about 200 mg/kg, about 500 mg/kg, about 1 g/kg, about 5 g/kg, about 10 g/kg, about 20 g/kg, about 50 g/kg, about 70 g/kg, about 90 g/kg or about 100 g/kg). A given dosage may be administered at various intervals, such as for example once a day, two or more times per day, two or more times per month, once per week, once every two weeks, once every three weeks, once a month, or once every two or more months. In certain embodiments, the administration dosage may change over the course of treatment. For example, in certain embodiments, the initial administration dosage may be higher than subsequent administration dosages. In certain embodiments, the administration dosage may vary over the course of treatment depending on the response of the subject.

Dosage regimens may be adjusted to provide the optimum desired response (e.g., a therapeutic response). For example, a single dose may be administered, or several divided doses may be administered over time.

In a further aspect, the present invention provides the use of a PEG-PBG copolymer in the preparation of a medicament for treating a disease. In certain embodiments, the medicament further comprises the active ingredient.

In another aspect, the present invention provides a method for preparing a medicament, comprising mixing the active ingredient with the PEG-PBG copolymer.

**Specific Examples**

Preferred examples of the present invention are set forth below, and it will be appreciated that these preferred examples are provided only to illustrate and explain the invention and are not to be interpreted as limiting it.

Unless otherwise explicitly indicated, all the PEG-PBG copolymers used in the following examples were those of formula I obtained from Advanced Polymer Materials Inc. (Canada).

**Example 1 Preparation of Paclitaxel/PEG-PBG Nanoparticles**

An organic phase was prepared by dissolving 40 mg of a PEG-PBG copolymer (n=45, m=20) and 8 mg of paclitaxel in 10 ml of acetone serving as a solvent, and 10 ml of water was added thereto as an aqueous phase. The organic phase was added dropwise to the aqueous phase at a rate of 5 ml/min with mechanical agitation at 300 rpm so that light blue nanoparticles were obtained. After agitation for 10 min, the mixture was transferred into a rotary evaporator, and acetone was removed by rotary evaporation at a vacuum degree of -0.1 MPa for 30 min, resulting in stable nanoparticles.

The nanoparticles were measured on a dynamic laser scatterometer (Beckman Coulter, LS 13320) to have an average particle size of 22.4 $\pm$ 1.6 nm and a coefficient of dispersion of 0.118. A particle size distribution and a transmission electron microscopy (TEM) image of the nanoparticles are shown in FIG. 1. The encapsulation efficiency of the nanoparticles was determined to be 86.4$\pm$3% using the method as detailed below in Example 7.

**Example 2 Preparation of Cabazitaxel/PEG-PBG Nanoparticles**

An organic phase was prepared by dissolving 40 mg of a PEG-PBG copolymer (n=45, m=20) and 4 mg of cabazitaxel in 10 ml of acetone serving as a solvent, and 10 ml of water was added thereto as an aqueous phase. The organic phase was added dropwise to the aqueous phase at a rate of 5 ml/min with mechanical agitation at 300 rpm so that light blue nanoparticles were obtained. After agitation for 10 min, the mixture was transferred into a rotary evaporator, and acetone was removed by rotary evaporation at a vacuum degree of -0.1 MPa for 30 min, resulting in stable, long-circulating nanoparticles.

The nanoparticles were measured on the dynamic laser scatterometer to have an average particle size of 40.44 ± 2.5 nm and a coefficient of dispersion of 0.158. The encapsulation efficiency of the nanoparticles was determined to be 90.4±3% using the method as detailed below in Example 7.

**Example 3 Preparation of Other PEG-PBG Nanoparticles**

Nanoparticles were prepared from paclitaxel, docetaxel and cabazitaxel, as active ingredients, and different PEG-PBG copolymers of formula I (including those (n=17, m=16), (n=17, m=20), (n=17, m=22), (n=45, m=10), (n=45, m=15), (n=45, m=20), (n=45, m=23), (n=45, m=25), (n=45, m=28), (n=50, m=70), (n=68, m=22), (n=68, m=27), (n=113, m=90)) and PEG-PBG copolymers of formula II (n1=45, m=46, n2=20) at different PEG-PBG copolymer-to-active ingredient ratios (including 5:1, 10:1 and 20:1) using a method similar to that of Examples 1 and 2. All the nanoparticles so prepared are considered to exhibit a particle size of less than 100 nm, a uniform particle size distribution and encapsulation efficiency of greater than 70%, and detail data of them are omitted.

**Example 4 Preparation of Paclitaxel/PEG-PBG Nanoparticles Involving Reverse Dropwise Addition**

An organic phase was prepared by dissolving 40 mg of a PEG-PBG copolymer (n=113, m=90) and 4 mg of paclitaxel in 10 ml of acetone serving as a solvent, and 10 ml of water was added thereto as an aqueous phase. The aqueous phase was added dropwise to the organic phase at a rate of 5 ml/min with mechanical agitation at 300 rpm so that nanoparticles were obtained. After agitation for 10 min, the mixture was transferred into a rotary evaporator, and acetone was removed by rotary evaporation at a vacuum degree of -0.1 MPa for 30 min, resulting in stable, long-circulating nanoparticles.

The nanoparticles were measured on the dynamic laser scatterometer to have an average particle size of 60.02 ± 3.1 nm and a coefficient of dispersion of 0.128. The encapsulation efficiency of the nanoparticles was determined to be 91.3±2% using the method as detailed below in Example 7.

**Example 5 Preparation of Paclitaxel/PEG-PBG Nanoparticles Involving Reverse Dropwise Addition and Film Formation by Spinning**

In 10 ml of acetone serving as a solvent, 40 mg of a PEG-PBG copolymer (n=50, m=70) and 4 mg of paclitaxel were dissolved, followed by the formation of a film through spinning at a temperature of 60 °C under reduced pressure. The film then underwent rotary evaporation in vacuum for 1 h and was further dried for 12 h in a vacuum oven. Following the drying process, it was hydrated with water contained in the rotary evaporator at 60 °C under atmospheric pressure for 30 min so that nanoparticles were obtained.

The nanoparticles were measured on the dynamic laser scatterometer to have an average particle size of 80.13 ± 2.7 nm and a coefficient of dispersion of 0.154. The encapsulation efficiency of the nanoparticles was determined to be 90.1±3.1% using the method as detailed below in Example 7.

**Example 6 Stability of Paclitaxel/PEG-PBG Nanoparticles**

The paclitaxel/PEG-PBG (n=45, m=20) nanoparticles prepared in Example 1 were diluted with a 0.9% sodium chloride injection so that paclitaxel was present at concentration of 1 mg/ml, followed by homogenization. The sample was then placed in a constant temperature oven at 25 and 4 °C and observed for the precipitation.

It was observed that the sample remained stable for more than 48 hours at 25 °C and for more than 36 hours at 4 °C. Therefore, the paclitaxel/PEG-PBG nanoparticles prepared in accordance with the present application are very stable.

**Example 7 Encapsulation Efficiency of Paclitaxel/PEG-PBG Nanoparticles**

High performance liquid chromatography (HPLC) was employed to analyze the amount of paclitaxel. HPLC conditions were as follows: column: Agilent C18; mobile phase: acetonitrile-wafer mixture (50:50, v/v); detection wavelength: 227 nm; flow rate: 1.0 ml/min; feed volume: 20 μl. Paclitaxel standard solutions with different concentrations ranging from 0.25 μg/ml to 50 μg/ml were analyzed under the above HPLC conditions. A peak area vs. concentration curve was fitted and a regression equation was developed.

A sample of the nanoparticle suspension prepared in accordance with the present application was first centrifuged at a low rate of 1,000 rpm for 10 min to get rid of crystals of the drug that were not encapsulated, and was then centrifuged at a high rate of 10,000 rpm for 30 min. The supernatant was aspirated away and the remainder was then

reconstituted with high-purity water and then dissolved in the same volume of added acetonitrile. The obtained solution was analyzed under the foregoing HPLC conditions for the amount of paclitaxel contained therein. Meanwhile, an intact sample of the nanoparticle suspension was dissolved in the same volume of acetonitrile and measured for the amount of contained paclitaxel under the same HPLC conditions.

The encapsulation efficiency was calculated according to the following equation:

$$\text{Encapsulation Efficiency (\%)} = \text{Amount Encapsulated in Nanoparticles / Total Amount} \times 100\%$$

Encapsulation efficiency of the nanoparticles prepared in accordance with the present application was averaged at 83-95%.

### Example 8 *In Vitro* Release of Paclitaxel/PEG-PBG Nanoparticles

*In vitro* release was evaluated using a dialysis bag diffusion method.

A dialysis bag was soaked in distilled water for several minutes. One end of the dialysis bag was twisted and tied into a knot, and the bag was washed thrice with distilled water introduced from the other end thereof. Nanoparticles prepared in accordance with Example 1 were diluted with distilled water to 10 ml, of which 1 ml was reserved as a blank and the remaining 9 ml was placed into the dialysis bag. After the dialysis bag was tightly closed, it was submerged in a 50-ml PBS buffer (pH 7.4, containing 0.2% Tween 80) and shaken on a shaking table at 100 rpm at 37 °C. 1-Ml samples were taken from the PBS buffer outside the dialysis bag at pre-set times, and each sampling was followed by the addition of fresh buffer to replace the sample volume. Each of the samples was mixed and homogenized with 1.0 ml of acetonitrile and analyzed under the same HPLC conditions as in Example 5 to determine the amounts of paclitaxel contained therein. Percentages of cumulatively released paclitaxel were calculated, and a release profile was plotted.

The results of *in vitro* release are shown in Fig. 2, which indicates that the nanoparticles of the present application allow smooth, sustained release of the active ingredient at a reduced rate.

### Example 9 Pharmacokinetics of Paclitaxel/PEG-PBG Nanoparticles

Eight SD rats weighing 250±20 g were randomly divided into two groups. Each rat in one group was injected with 2 ml of Taxol via the tail vein, and each rat in the other group was injected with 2 ml of an aqueous solution of the paclitaxel/PEG-PBG nanoparticles prepared in Example 1 (1 mg/ml). 0.5-Ml blood samples were then collected from the orbital cavity of each rat at 5 minutes, 15 minutes, 30 minutes, 1 hour, 2 hours, 4 hours, 7 hours, and 24 hours after the injection in heparin tubes. Each blood sample was centrifuged at 4,000 rpm for 10 min, and 200 μL of the plasma was collected in a centrifuge tube, followed by addition thereto of 50 μL of an internal standard and vortexing. After 350 μL of acetonitrile was further added, the plasma was vortexed for 2 min and centrifuged at 13,000 rpm for 10 min. The supernatant was taken, membrane-filtered and analyzed under the same HPLC conditions as in Example 5. The resulting pharmacokinetic profiles are shown in Figure 3.

### Example 10 Cellular uptake of FITC-Loaded PEG-PBG Nanoparticles

BEL-7402 cells in the logarithmic growth phase were seeded in a well plate for confocal microscopy (repeated thrice) by adding 2 ml of a suspension of the cells to each well so that the cells were present at a concentration of $6\times10^5$ per cell, and cultured overnight to allow their adhesion. After 200 μL of FITC-loaded PEG-PBG (n=45, m=20) nanoparticles (with a FITC concentration of 100 μg/ml) was added, the cells were incubated respectively for 2 and 4 hours. Subsequently, the cells were gently washed twice with a PBS buffer and then fixed for 20 min with 300 μL of a 4% paraformaldehyde solution (v/v) added to each well. After the paraformaldehyde was removed, the cells were stained for 5 min with 300μL of a DAPI staining solution (5 μg/ml) added to each well. After washed twice with a PBS buffer, the cells were covered with 300μL of a PBS buffer added to each well and imaged using a confocal microscope. Results of cellular uptake of the FITC-loaded EB nanoparticles are shown in Fig. 4.

### Example 11 *In Vivo* Imaging with DiR-Loaded PEG-PBG Nanoparticles

During the preparation of nanoparticles, DiR dye was encapsulated (in place of the active ingredient) to prepare DiR-loaded PEG-PBG (n=45, m=20) nanoparticles (DIR-NPs) with a DiR concentration of 100 μg/ml. A549 tumor-bearing nude mice, each injected with 200 μL of a solution of the DiR-NPs via the tail vein, were anesthetized respectively 2, 8 and 24 hours after the injection and then fluorescence-imaged with an *in vivo* animal imaging system. At last, the mice

were killed and their organs or tissues including the hearts, livers, spleens, lungs, kidneys and tumors were immediately removed and fluorescence-imaged with the *in vivo* animal imaging system. The fluorescence imaging was carried out at an excitation wavelength of 730 nm and an emission wavelength of 790 nm with an exposure time of 1 min. X-ray imaging was further performed with an exposure time of 30 s. The obtained images were analyzed using the software Kodak MI In Vivo Fx Pro, in which the fluorescence images were superimposed with the X-ray images and pseudo-colors were applied to allow determination of fluorescence distributions in the organs based on the X-ray images. The resulting images of the nude mice and their tissues are shown in Fig. 5.

**Example 12 *In Vivo* Pharmacodynamics of Paclitaxel/PEG-PBG Nanoparticles in Animal Models**

A comparison was made between paclitaxel/PEG-PBG nanoparticles prepared in Example 1 and paclitaxel injections used in the current clinical practice in terms of effectiveness in inhibiting the growth of subcutaneous tumors in mouse models established by inoculating A549 (lung cancer cell line) cells.

The results showed that the paclitaxel/PEG-PBG nanoparticles had a good inhibitory effect on the growth of the subcutaneous tumors in the models. Compared to the injection formulations (with a paclitaxel dosage of 10 mg/kg), the PEG-PBG nanoparticle formulation (with a paclitaxel dosage of 10 mg/kg) was less toxic, had a longer lasting efficacy and dispensed with the need for frequent administration over a long time. The results of tumor growth inhibition and body weight variations in the nude mice are shown in Fig. 6.

## Claims

**1.** A composition, comprising an active pharmaceutical ingredient and a polyethylene glycol-polybutylene glycol (PEG-PBG) copolymer, wherein the composition is in a nanoparticle form and the active pharmaceutical ingredient is a hydrophobic substance, wherein the PEG-PBG copolymer is of formula I, II or III

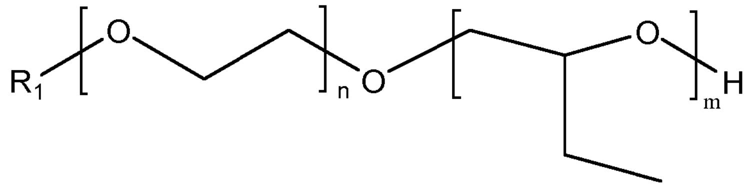

I

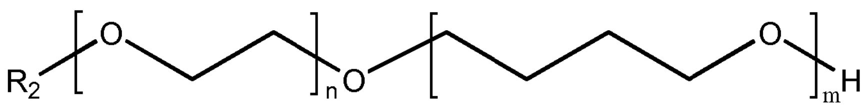

II

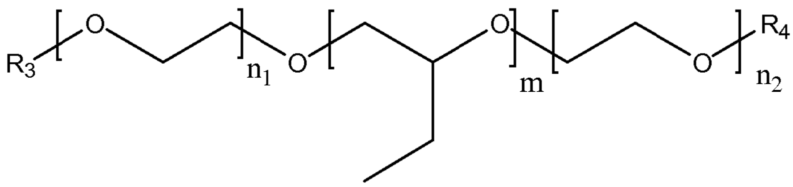

III

, in which n, n1 and n2 each independently range from 1 to 3000, m ranges from 1 to 1500, and $R_1$, $R_2$, $R_3$ and $R_4$ are each independently selected from H and $C_1$-$C_3$ alkyl groups.

**2.** The composition according to claim 1, wherein the PEG-PBG copolymer has a molecular weight of 0.1 K-300 K.

**3.** The composition according to claim 1, wherein the nanoparticles have a particle size of 10-500 nm, preferably a particle size of 10-100 nm.

**4.** The composition according to claim 1, wherein the active pharmaceutical ingredient is selected from anti-neoplastic agents, antibiotic agents, cardiovascular agents, anti-diabetic agents and non-steroidal anti-inflammatory agents, the active pharmaceutical ingredient preferably being paclitaxel and its derivatives, preferably paclitaxel, docetaxel or cabazitaxel.

**5.** The composition according to claim 1, wherein the active pharmaceutical ingredient and the PEG-PBG copolymer are present in a ratio by weight of 0.01-1, preferably in a ratio by weight of 0.1-0.3.

**6.** The composition according to claim 1, wherein the composition further comprises other polymers.

**7.** A method of preparing the composition as defined in any one of claims 1 to 6, comprising the steps of:

(a) dissolving the PEG-PBG copolymer and the active pharmaceutical ingredient in an organic solvent, wherein the active pharmaceutical ingredient is a hydrophobic substance;
(b) adding the organic phase to an aqueous solution to form an oil-water mixture; and
(c) removing the organic solvent from the oil-water mixture under reduced pressure.

**8.** The method according to claim 7, wherein step (b) further includes treating the oil-water mixture with a low shear force, preferably with agitation.

**9.** The method according to claim 7, wherein the method further comprises step (d) in which the product resulting from step (c) is dried, preferably dried by lyophilization.

**10.** The method according to claim 7, wherein the organic solvent comprises tetrahydrofuran, 1,4-dioxane, dimethyl sulfoxide, acetone, N,N-dimethylmethanamide or a mixture thereof.

**11.** The method according to claim 7, wherein the organic phase and aqueous phase are present in a ratio of 1:10-20:1, preferably in a ratio of from 0.5:1 to 2:1.

**12.** The composition as defined in any one of claims 1 to 6 for use in a method for relieving, treating, or preventing a disease, preferably for relieving, treating, or preventing a cancer.

**13.** Use of a polyethylene glycol-polybutylene glycol (PEG-PBG) copolymer in the preparation of a medicament for treating a disease, wherein the composition is in a nanoparticle form and the active pharmaceutical ingredient is a hydrophobic substance, wherein the PEG-PBG copolymer is of formula I, II or III

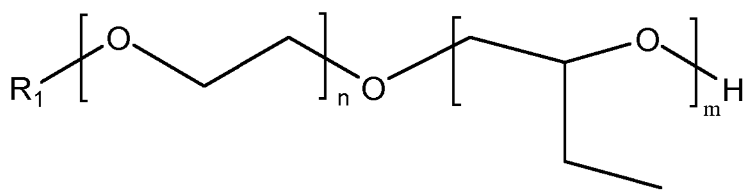

I

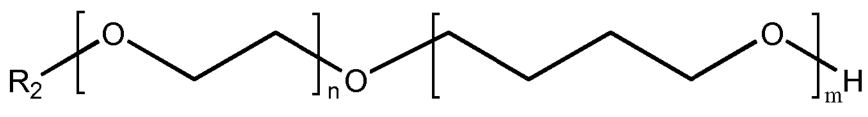

II

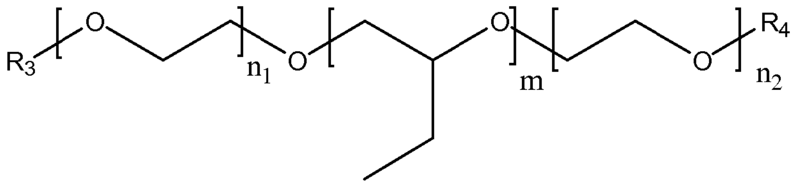

III

, in which n, n1 and n2 each independently range from 1 to 3000, m ranges from 1 to 1500, and $R_1$, $R_2$, $R_3$ and $R_4$ are each independently selected from H and $C_1$-$C_3$ alkyl groups.

**14.** A method of preparing a medicament, comprising the steps of:

(a) dissolving a PEG-PBG copolymer and an active pharmaceutical ingredient in an organic solvent, wherein the active pharmaceutical ingredient is a hydrophobic substance;
(b) adding an organic phase to an aqueous solution to form an oil-water mixture; and
(c) removing the organic solvent from the oil-water mixture under reduced pressure,

wherein the PEG-PBG copolymer is of formula I, II or III

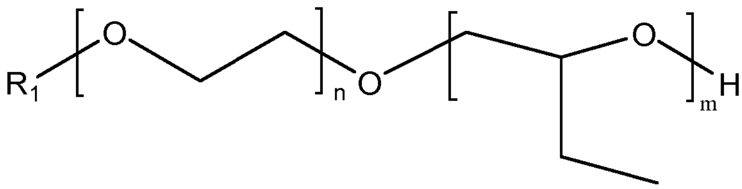

I

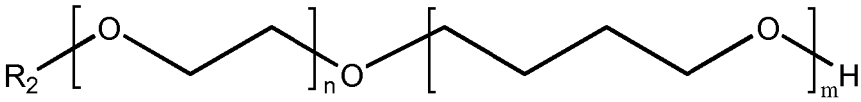

II

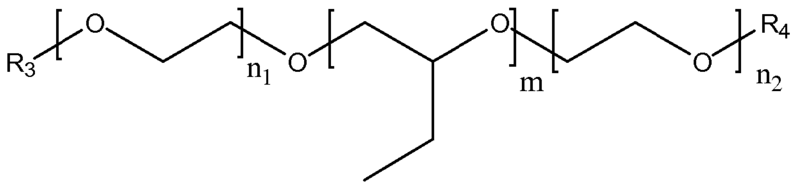

III

, in which n, n1 and n2 each independently range from 1 to 3000, m ranges from 1 to 1500, and $R_1$, $R_2$, $R_3$ and $R_4$ are each independently selected from H and $C_1$-$C_3$ alkyl groups.

## Patentansprüche

**1.** Zusammensetzung, einen pharmazeutischen Wirkstoff und ein Polyethylen-Glykol-Polybutylen-Glykol (PEG-PBG) Copolymer umfassend, wobei die Zusammensetzung in einer Nanopartikelform ist und der pharmazeutische Wirkstoff eine wasserabweisende Substanz ist, wobei das PEG-PBG Copolymer von der Formel I, II oder III ist

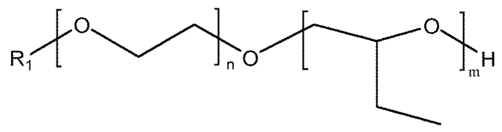

I

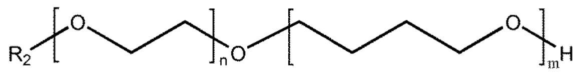

II

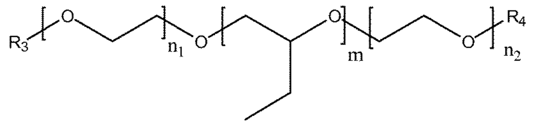

III

in der n, n1 und n2 jeweils unabhängig von 1 bis 3000 reichen, m von 1 bis 1500 reicht, und $R_1$, $R_2$, $R_3$ und $R_4$ jeweils unabhängig aus H und $C_1$-$C_3$ Alkyl-Gruppen ausgewählt sind.

**2.** Zusammensetzung nach Anspruch 1, wobei das PEG-PBG Copolymer ein Molekulargewicht von 0,1 K bis 300 K aufweist.

**3.** Zusammensetzung nach Anspruch 1, wobei die Nanopartikel eine Partikelgröße von 10 bis 500 nm, vorzugsweise eine Partikelgröße von 10 bis 100 nm aufweisen.

**4.** Zusammensetzung nach Anspruch 1, wobei der pharmazeutische Wirkstoff aus anti-neoplastischen Mitteln, Antibiotika, kardiovaskulären Stoffen, Anti-Diabetes Stoffen, nichtsteroidalen entzündungshemmenden Stoffen ausgewählt sind, wobei der pharmazeutische Wirkstoff vorzugsweise Paclitaxel und dessen Derivate, vorzugsweise Paclitaxel, Docetaxel oder Cabazitaxel ist.

**5.** Zusammensetzung nach Anspruch 1, wobei der pharmazeutische Wirkstoff und das PEG-PBG Copolymer in einem Gewichtsverhältnis von 0,01 bis 1, vorzugsweise in einem Gewichtsverhältnis von 0,1 bis 0,3 vorhanden sind.

**6.** Zusammensetzung nach Anspruch 1, wobei die Zusammensetzung weiter andere Polymere umfasst.

**7.** Verfahren zur Herstellung einer Zusammensetzung nach einem der Ansprüche 1 bis 6, die folgenden Schritte umfassend:

(a) Auflösen des PEG-PBG Copolymers und des pharmazeutischen Wirkstoffs in einem organischen Lösungsmittel, wobei der pharmazeutische Wirkstoff eine wasserabweisende Substanz ist;
(b) Beigeben der organischen Phase zu einer wässrigen Lösung zur Bildung eines Öl-Wasser-Gemisches; und
(c) Entfernen des organischen Lösungsmittels aus dem Öl-Wasser-Gemisch unter verringertem Druck.

**8.** Verfahren nach Anspruch 7, wobei Schritt (b) weiter das Behandeln des Öl-Wasser-Gemisches mit einer niedrigen Scherkraft, vorzugsweise durch Schütteln beinhaltet.

**9.** Verfahren nach Anspruch 7, wobei das Verfahren weiter Schritt (d) umfasst, bei dem das aus Schritt (c) resultierende Produkt getrocknet, vorzugsweise durch Lyophilisieren getrocknet wird.

**10.** Verfahren nach Anspruch 7, wobei das organische Lösungsmittel Tetrahydrofuran, 1,4-Dioxan, Dimethyl Sulfoxid, Aceton, N,N-Dimethylmethanamid oder ein Gemisch daraus umfasst.

**11.** Verfahren nach Anspruch 7, wobei die organische Phase und wässrige Phase in einem Verhältnis von 1;10-20:1, vorzugsweise in einem Verhältnis von 0,5:1-2:1 vorhanden sind.

**12.** Zusammensetzung nach einem der Ansprüche 1 bis 6 zur Verwendung bei einem Verfahren zum Abschwächen, Behandeln, oder Vorbeugen einer Erkrankung, vorzugsweise zum Abschwächen, Behandeln, oder Vorbeugen von Krebs.

**13.** Verwendung eines Polyethylen-Glykol-Polybutylen-Glykol (PEG-PBG) Copolymers bei der Herstellung eines Medikaments zur Behandlung einer Erkrankung, wobei die Zusammensetzung in einer Nanopartikelform ist und der pharmazeutische Wirkstoff eine wasserabweisende Substanz ist, wobei das PEG-PBG Copolymer von der Formel I, II oder III ist

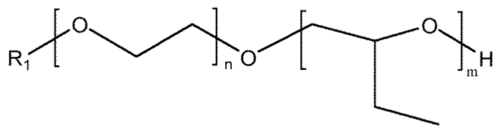

I

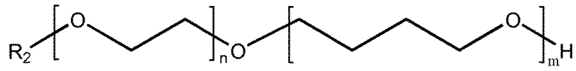

II

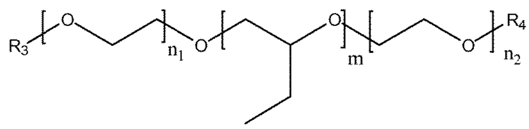

III

in der n, n1 und n2 jeweils unabhängig von 1 bis 3000 reichen, m von 1 bis 1500 reicht, und $R_1$, $R_2$, $R_3$ und $R_4$ jeweils unabhängig aus H und $C_1$-$C_3$ Alkyl-Gruppen ausgewählt sind.

**14.** Verfahren zur Herstellung eines Medikaments, die folgenden Schritte umfassend:

(a) Auflösen des PEG-PBG Copolymers und des pharmazeutischen Wirkstoffs in einem organischen Lösungsmittel, wobei der pharmazeutische Wirkstoff eine wasserabweisende Substanz ist;
(b) Beigeben einer organischen Phase zu einer wässrigen Lösung zur Bildung eines Öl-Wasser-Gemisches; und
(c) Entfernen des organischen Lösungsmittels aus dem Öl-Wasser-Gemisch unter verringertem Druck,

wobei das PEG-PBG Copolymer von der Formel I, II oder III ist

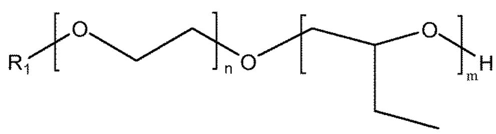

I

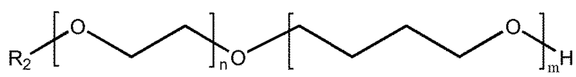

II

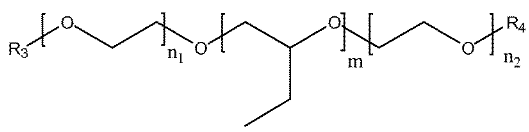

III

in der n, n1 und n2 jeweils unabhängig von 1 bis 3000 reichen, m von 1 bis 1500 reicht, und $R_1$, $R_2$, $R_3$ und $R_4$ jeweils unabhängig aus H und $C_1$-$C_3$ Alkyl-Gruppen ausgewählt sind.

## Revendications

**1.** Composition, comprenant un principe pharmaceutique actif et un copolymère de polyéthylène glycol-polybutylène glycol (PEG-PBG), dans laquelle la composition est sous une forme nanoparticulaire et le principe pharmaceutique actif est une substance hydrophobe, dans laquelle le copolymère de PEG-PBG est de formule I, II ou III

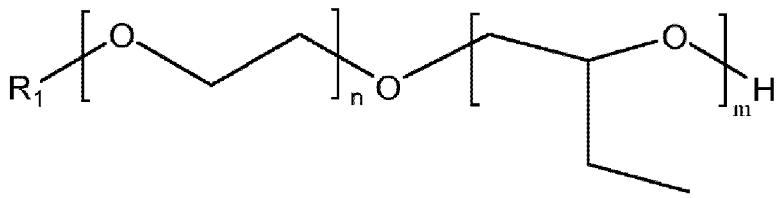

I

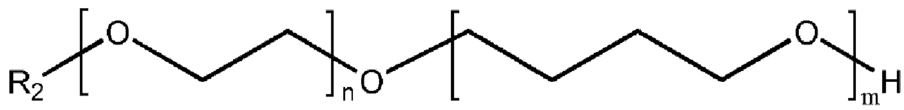

II

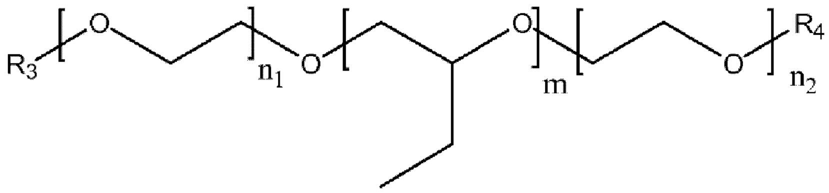

III

dans lesquelles n, n1 et n2 sont chacun indépendamment dans la plage de 1 à 3000, m est dans la plage de 1 à 1500, et $R_1$, $R_2$, $R_3$ et $R_4$ sont chacun indépendamment sélectionnés parmi H et des groupes alkyle en $C_1$-$C_3$.

**2.** Composition selon la revendication 1, dans laquelle le copolymère de PEG-PBG a un poids moléculaire de 0,1 K à 300 K.

**3.** Composition selon la revendication 1, dans laquelle les nanoparticules ont une taille des particules de 10 à 500 nm, de préférence une taille des particules de 10 à 100 nm.

**4.** Composition selon la revendication 1, dans laquelle le principe pharmaceutique actif est sélectionné parmi les agents antinéoplasiques, les agents antibiotiques, les agents cardiovasculaires, les agents antidiabétiques, et les agents anti-inflammatoires non stéroïdiens, le principe pharmaceutiquement actif étant de préférence le paclitaxel et ses dérivés, de préférence le paclitaxel, le docétaxel ou le cabazitaxel.

**5.** Composition selon la revendication 1, dans laquelle le principe pharmaceutique actif et le copolymère de PEG-PBG sont présents en un rapport en poids de 0,01 à 1, de préférence en un rapport en poids de 0,1 à 0,3.

**6.** Composition selon la revendication 1, dans laquelle la composition comprend en outre d'autres polymères.

**7.** Procédé de préparation de la composition selon l'une quelconque des revendications 1 à 6, comprenant les étapes de :

(a) dissolution du copolymère de PEG-PBG et du principe pharmaceutique actif dans un solvant organique,

dans lequel le principe pharmaceutique actif est une substance hydrophobe ;
(b) ajout de la phase organique à une solution aqueuse pour former un mélange huile-eau ; et
(c) retrait du solvant organique du mélange huile-eau sous pression réduite.

**8.** Procédé selon la revendication 7, dans lequel l'étape (b) inclut en outre le traitement du mélange huile-eau avec une faible force de cisaillement, de préférence avec agitation.

**9.** Procédé selon la revendication 7, dans lequel le procédé comprend en outre l'étape (d) dans laquelle le produit résultant de l'étape (c) est séché, de préférence séché par lyophilisation.

**10.** Procédé selon la revendication 7, dans lequel le solvant organique comprend du tétrahydrofurane, du 1,4-dioxane, du diméthylsulfoxyde, de l'acétone, du N,N-diméthylméthanamide ou un mélange de ceux-ci.

**11.** Procédé selon la revendication 7, dans lequel la phase organique et la phase aqueuse sont présentes en un rapport de 1:10 à 20:1, de préférence en un rapport de 0,5:1 à 2:1.

**12.** Composition selon l'une quelconque des revendications 1 à 6 pour son utilisation dans un procédé de soulagement, de traitement ou de prévention d'une maladie, de préférence de soulagement, de traitement ou de prévention d'un cancer.

**13.** Utilisation d'un copolymère de polyéthylène glycol-polybutylène glycol (PEG-PBG), dans la préparation d'un médicament pour le traitement d'une maladie, dans laquelle la composition est sous une forme nanoparticulaire et le principe pharmaceutique actif est une substance hydrophobe, dans laquelle le copolymère de PEG-PBG est de formule I, II ou III

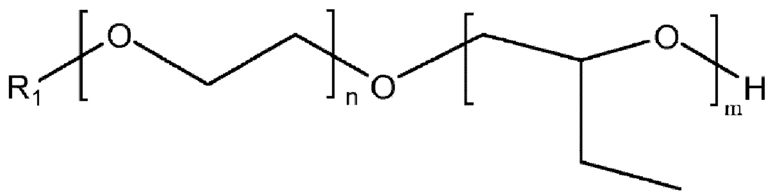

I

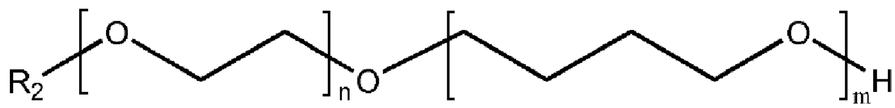

II

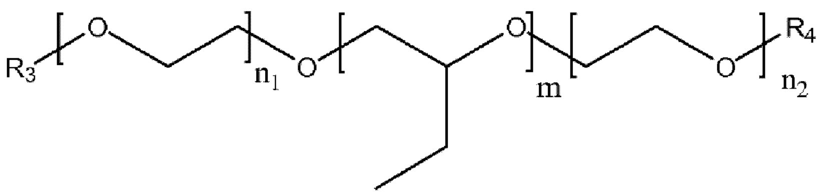

III

dans lesquelles n, n1 et n2 sont chacun indépendamment dans la plage de 1 à 3000, m est dans la plage de 1 à 1500, et $R_1$, $R_2$, $R_3$ et $R_4$ sont chacun indépendamment sélectionnés parmi H et des groupes alkyle en $C_1$-$C_3$.

**14.** Procédé de préparation d'un médicament, comprenant les étapes de :

(a) dissolution d'un copolymère de PEG-PBG et d'un principe pharmaceutique actif dans un solvant organique, dans lequel le principe pharmaceutique actif est une substance hydrophobe ;
(b) ajout d'une phase organique à une solution aqueuse pour former un mélange huile-eau ; et

(c) retrait du solvant organique du mélange huile-eau sous pression réduite, dans lequel le copolymère de PEG-PBG est de formule I, II ou III

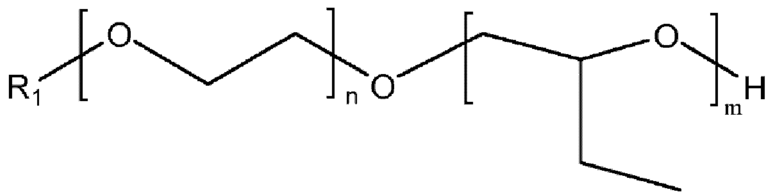

I

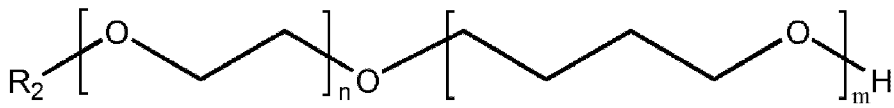

II

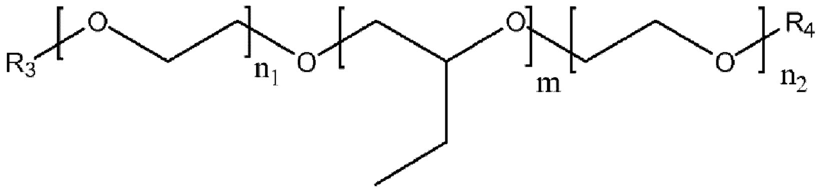

III

dans lesquelles n, n1 et n2 sont chacun indépendamment dans la plage de 1 à 3000, m est dans la plage de 1 à 1500, et $R_1$, $R_2$, $R_3$ et $R_4$ sont chacun indépendamment sélectionnés parmi H et des groupes alkyle en $C_1$-$C_3$.

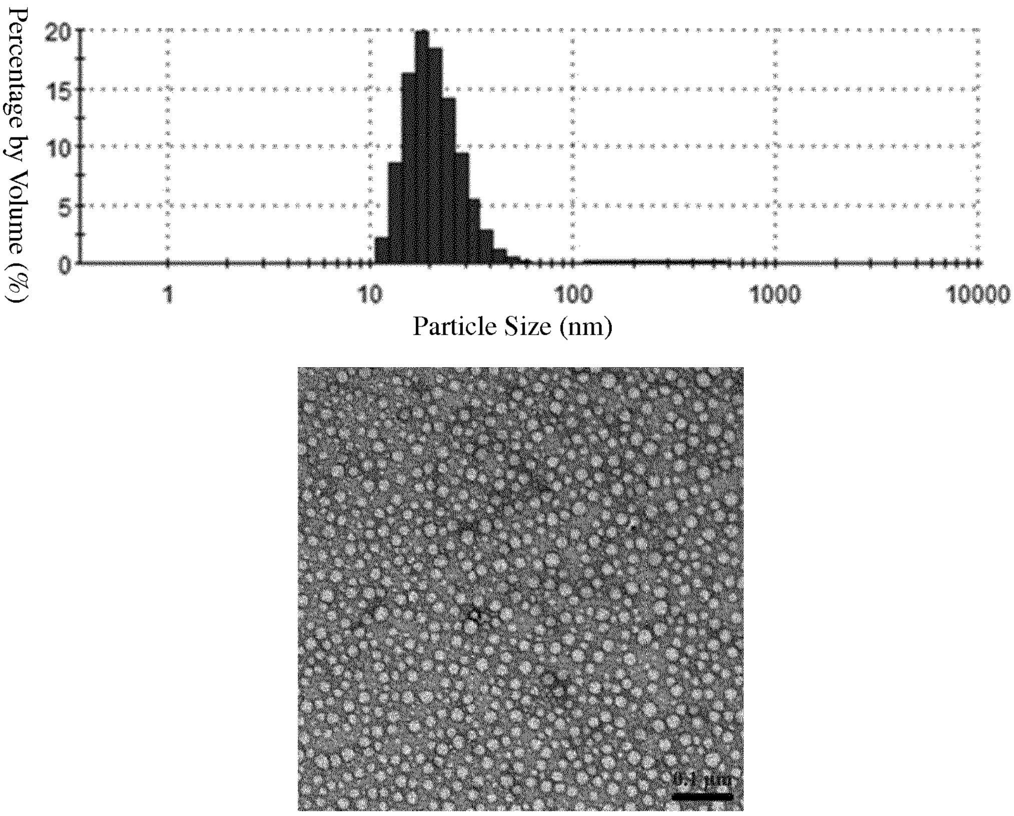
Percentage by Volume (%)
0
5
10
15
20
1
10
100
1000
10000
Particle Size (nm)

Fig. 1

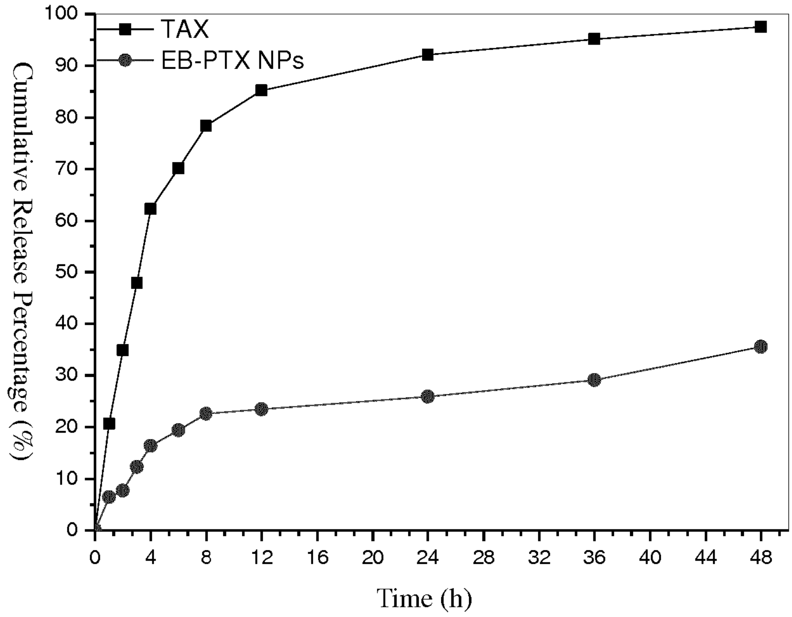
TAX
EB-PTX NPs
Cumulative Release Percentage (%)
0
10
20
30
40
50
60
70
80
90
100
0
4
8
12
16
20
24
28
32
36
40
44
48
Time (h)

Fig. 2

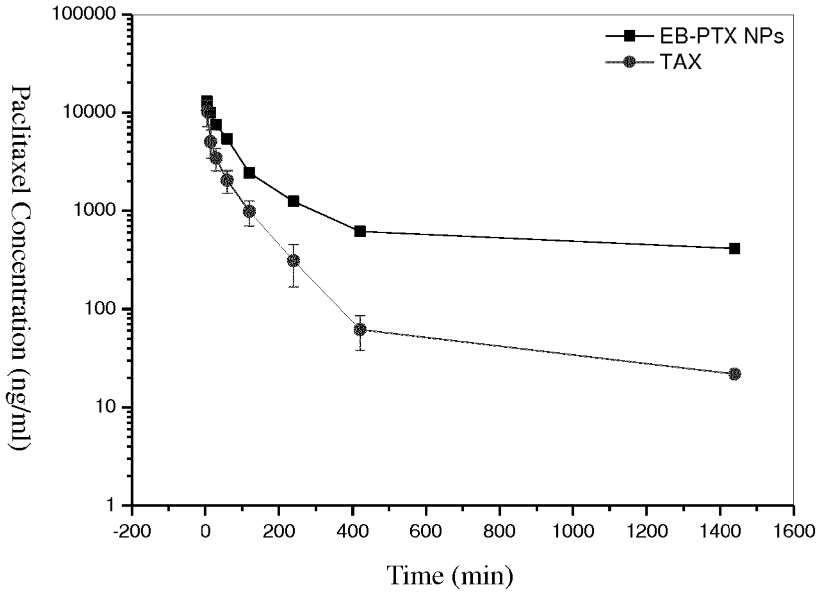
100000
10000
1000
100
10
1
-200
0
200
400
600
800
1000
1200
1400
1600
Paclitaxel Concentration (ng/ml)
Time (min)
EB-PTX NPs
TAX

Fig. 3

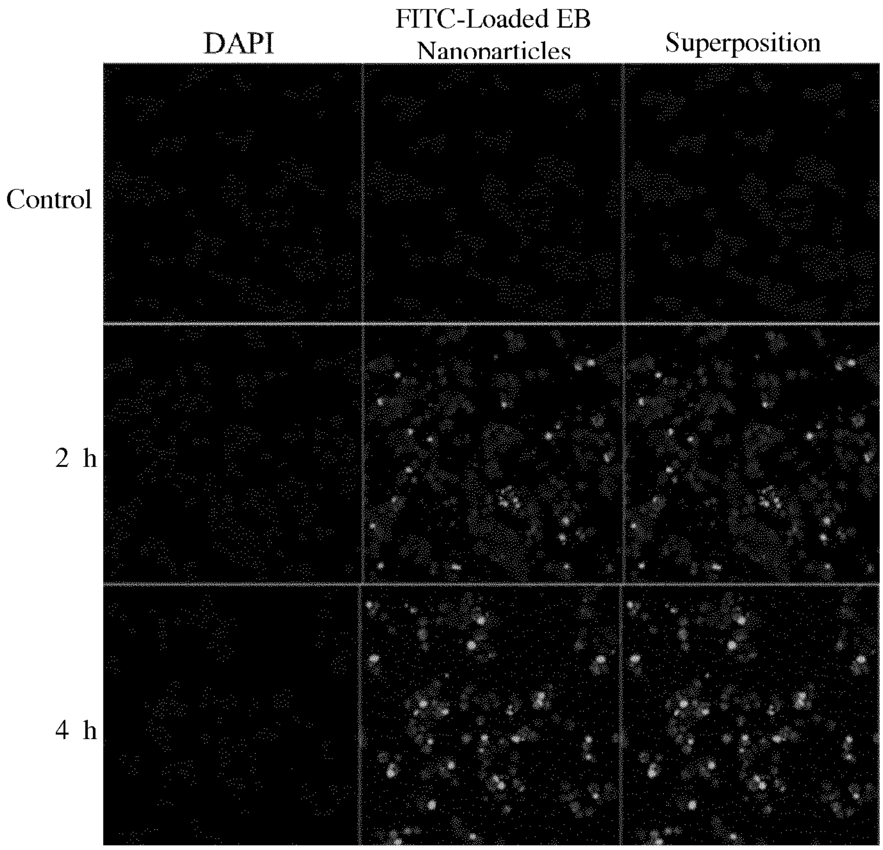
DAPI
FITC-Loaded EB Nanoparticles
Superposition
Control
2 h
4 h

Fig. 4

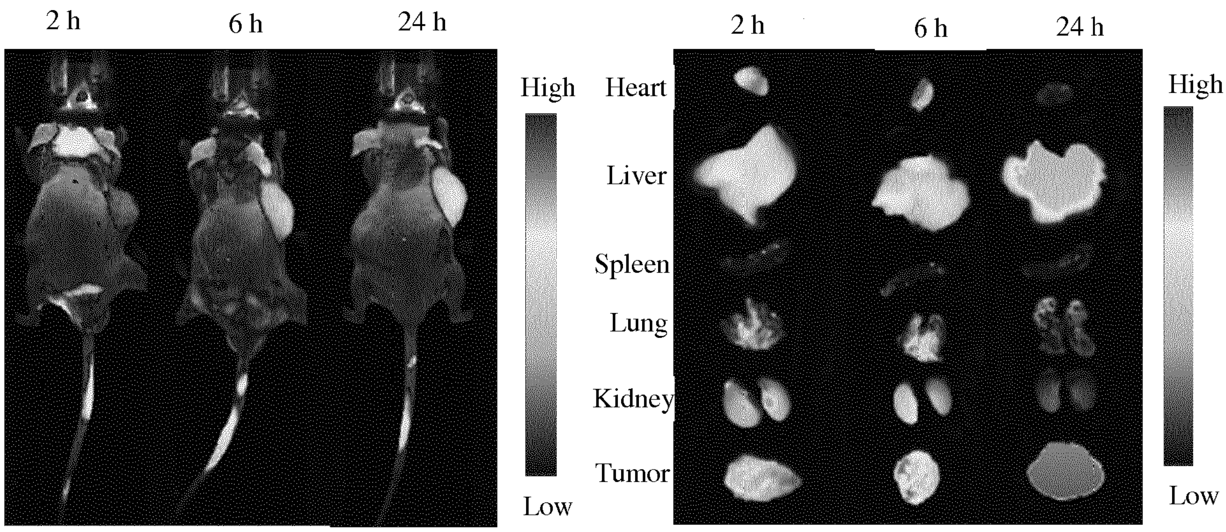
2 h
6 h
24 h
High
Low
2 h
6 h
24 h
Heart
Liver
Spleen
Lung
Kidney
Tumor
High
Low

Fig. 5

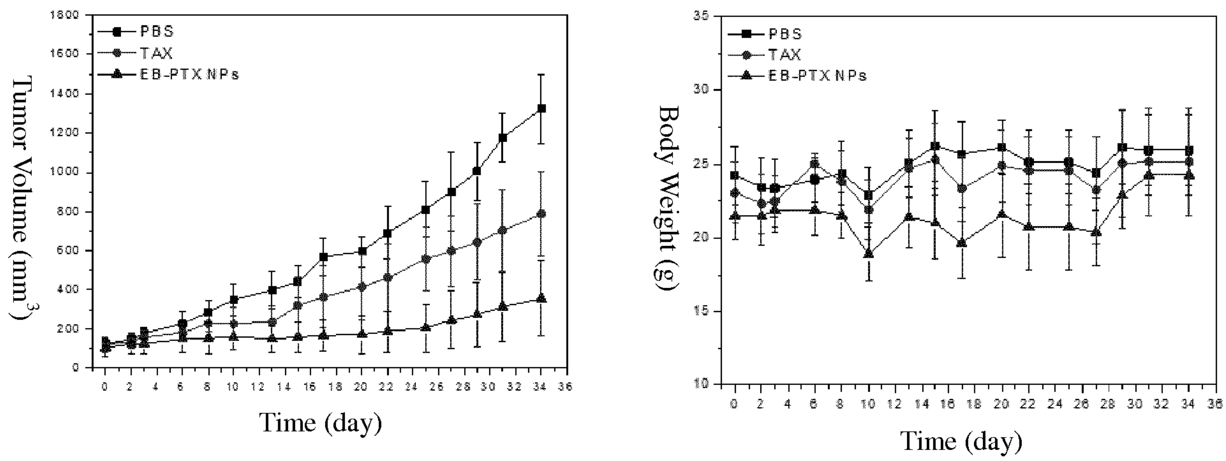
PBS
TAX
EB-PTX NPs
Tumor Volume (mm$^3$)
Time (day)
PBS
TAX
EB-PTX NPs
Body Weight (g)
Time (day)

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102167794 A
- CN 103263672 A
- CN 104136012 A
- US 2004009229 A1